# EUROPÄISCHE PATENTSCHRIFT

(11) **EP 2 826 518 B1**
(45) Veröffentlichungstag und Bekanntmachung des Hinweises auf die Patenterteilung: **16.05.2018**
(21) Anmeldenummer: 13003581.9
(22) Anmeldetag: 16.07.2013
(51) Int. Cl.: A61M 37/00

(54) **Nadelmodul und Gerät zum Anstechen der Haut**
Needle module and apparatus for piercing the skin
Module d'aiguille et appareil destiné à piquer la peau

(43) Veröffentlichungstag der Anmeldung: 21.01.2015
(73) Patentinhaber: Rene La Fontaine, 76863 Herxheim (DE); Hoffmann, Holger, 20357 Hamburg (DE)
(72) Erfinder: LA Fontaine, Helmut, 29600 Marbella (ES)
(74) Vertreter: Zellentin & Partner mbB Patentanwälte

(56) Entgegenhaltungen:
- EP-A2- 2 420 265
- DE-A1- 10 343 590
- US-B1- 6 345 553

## Beschreibung

Die vorliegende Erfindung betrifft ein Nadelmodul, welches mit einer Antriebsvorrichtung zu einem Gerät für ein Anstechen der Haut, z.B. für Permanent Make-up oder Tätowierungen, koppelbar ist.

Beim Pigmentieren der Haut, das sich in das Tätowieren und das Permanent Make-up unterteilen lässt, wird eine Vielzahl an spezifischen Farbpigmenten mit einer speziell geformten Nadel oder einem Nadelsystem aus einer Mehrzahl von Nadeln durch Anstechen der Haut in die Schichten des Hautorgans eingetragen bzw. transportiert. Außerdem können mit analogen Vorrichtungen in der Medizin Wirkstoffe in die Haut eingebracht werden.

Ein typisches Handgerät zum Anstechen der Haut besteht aus einer Antriebseinheit und einem lösbar damit verbundenen Nadelmodul. Die Antriebseinheit umfasst in der Regel einen Motor, ein Getriebe und einen Abtrieb zum Erzeugen einer oszillatorischen Hin- und Her-Bewegung der Nadel. Das Nadelmodul umfasst die Nadel bzw. das Nadelsystem, welche(s) in einem Nadelhalter aufgenommen ist. Der Nadelhalter ist mit der Antriebseinheit verbindbar. Die Antriebseinheit enthält dazu üblicherweise eine Schubstange mit einer speziellen Aufnahme, in die der Nadelhalter, abhängig vom Hersteller, eingeschoben, aufgesteckt und/oder verklemmt wird.

Die Nadel kann eine spezifisch geformte, einzelne Nadel oder ein Nadelsystem, d.h. eine Zusammenstellung von mehreren Nadeln sein. Die Nadel führt während des Anstechens eine oszillatorische Hin- und Herbewegung mit einer vom Bediener einstellbaren Geschwindigkeit und Einstechtiefe aus. Der Einstechvorgang erfolgt somit in einer definierten Art und Weise. Ziel ist es, einen vorgegebenen Farbstoff (oder Wirkstoff) durch oszillatorisches Anstechen in tiefer liegende Hautschichten einzubringen. Die Nadel wird entweder regelmäßig in den Farbstoff bzw. Wirkstoff eingetaucht oder der Farbstoff bzw. Wirkstoff ist in einem Reservoir im Nadelmodul angeordnet.

Zu Beginn einer Behandlung wird eine - in der Regel sterile - Nadel mit der Antriebseinheit des Geräts verbunden. Nach Beendigung der Behandlung sollte die Nadel mit Nadelführung entsorgt werden.

Solche Geräte, insbesondere Handgeräte, zum Anstechen der Haut sind an sich bekannt und beispielsweise in EP 1 743 673 A1 und EP 1 872 823 A1 beschrieben.

Gemäß EP 1 743 673 A1 soll die Widerstandskraft beim Anstechen gemessen werden und über ein mit dem Handgerät verbundenes Steuergerät auf eine Änderung der Widerstandskraft mit einer Änderung bei der Repetierfrequenz des Einstechens reagiert werden.

Die EP 1 872 823 A1 schlägt vor, Kennungsmittel an dem Nadelmodul vorzusehen, welche durch eine Auswertevorrichtung an der Antriebseinheit ausgewertet werden. Dadurch soll vor allem die Kopplung eines mit der Antriebseinheit inkompatiblen Nadelmoduls verhindert werden. Zusäzlich können aber auch Informationen über zulässige Betriebsparameter des Nadelmoduls eingebracht und ausgewertet werden. Weiterhin erlauben es Kennungsmittel in Form von elektronischen Datenspeichern, z.B. RFID-Chips, eine unzulässige Mehrfachnutzung eines Nadelmoduls zu blockieren. Eine solche Sicherung ist sehr wünschenswert, jedoch erfordern elektronische Kennungsmittel eine entsprechende Auswertevorrichtung, wodurch die Geräte erheblich teurer werden. Außerdem sind elektronische Systeme aufwändig, störanfällig und manipulierbar. Es besteht daher Bedarf an einfachen Möglichkeiten zur Sicherung.

Ein weiteres Problem ergibt sich beim Abnehmen und Entsorgen der Nadelmodule. Im Gegensatz zu frischen Nadelmodulen ist nach dem Gebrauch die Nadel nicht durch eine Kappe und/oder Verpackung abgedeckt, so dass ein Verletzungsrisiko besteht. Sowohl ein Selbststechen des Bedieners als auch eine Verletzung anderer Personen ist leicht möglich. Siehe auch DE10343590, US6345553 und EP2420265 Überraschend wurde nun gefunden, dass ein Nadelmodul mit Hilfe einer mechanischen Sicherungsvorrichtung, welche ein Ausfahren der Nadel aus dem Nadelmodul beim und nach dem Abnehmen des Nadelmoduls von der Antriebseinheit irreversibel blockiert, die bestehenden Probleme überwindet.

Die in Anspruch 1 definierte Erfindung löst daher die obigen Aufgaben durch ein Nadelmodul, umfassend ein Anstechmittel an einer Halterung, welche mit einer Antriebseinheit lösbar koppelbar ist, so dass durch eine von der Antriebseinheit auf die Halterung ausgeübte Antriebskraft eine repetierende Schubbewegung zum Ein- und Ausfahren des Anstechmittels übertragen wird, wobei die Halterung eine Sicherungsvorrichtung aufweist, welche das Anstechmittel nach dem Lösen des Nadelmoduls von der Antriebseinheit so mechanisch irreversibel blockiert, dass es nicht mehr aus dem Nadelmodul ausgefahren werden kann.

Die Erfindung betrifft außerdem ein Gerät, insbesondere ein Handgerät, zum Anstechen der Haut, umfassend eine Antriebseinheit und ein Nadelmodul, wobei die Antriebseinheit einen Motor und Kopplungsmittel aufweist, und das Nadelmodul ein Anstechmittel an einer Halterung umfasst, welche mit der Antriebseinheit lösbar koppelbar ist, so dass durch eine von der Antriebseinheit auf die Halterung ausgeübte Antriebskraft eine repetierende Schubbewegung zum Ein-und Ausfahren des Anstechmittels übertragen wird, wobei die Halterung eine Sicherungsvorrichtung aufweist, welche das Anstechmittel nach dem Lösen des Nadelmoduls von der Antriebseinheit so mechanisch irreversibel blockiert, dass es nicht mehr aus dem Nadelmodul ausgefahren werden kann.

Somit gewährleisten Nadelmodul und Gerät gemäß der Erfindung, dass ein Nadelmodul nicht versehentlich mehrfach benutzt wird. Eine solche Mehrfachnutzung birgt das Risiko einer Kreuzkontamination, indem Krankheitserreger von einem Behandeltem zum nächsten übertragen werden.

Ein großer Vorteil der Erfindung ist, dass beim Abnehmen und Entsorgen des Nadelmoduls das Verletzungsrisiko erheblich verringert wird. Ein zufälliges Selbststechen oder Stechen anderen Personen im Umfeld an einer frei zugänglich liegenden, verschmutzten Nadel wird nahezu ausgeschlossen.

Das erfindungsgemäße Nadelmodul weist als Anstechmittel eine Nadel auf, oder ein Nadelsystem mit zwei, drei oder noch mehr Nadeln. Die Nadeln werden in an sich bekannter Weise ausgebildet. Es ist zweckmäßig, wenn das Anstechmittel vor dem Gebrauch mit einer Schutzkappe abgedeckt ist, die erst nach dem Ankoppeln des Nadelmoduls an die Antriebseinheit abgezogen wird. Eine Schutzkappe ist besonders bevorzugt, wenn das Anstechmittel vor dem Gebrauch aus dem Nadelmodul herausragt. Außerdem ist es bevorzugt, wenn das Nadelmodul sterilisiert und/oder bis zum Gebrauch in einer Verpackung gelagert wird.

Weiterhin weist das Nadelmodul eine Halterung für das Anstechmittel auf, welche mit der Antriebseinheit in wiederlösbarer Art koppelbar ist. Über die Halterung werden die von der Antriebseinheit erzeugten repetierenden Schubbewegungen auf das Anstechmittel übertragen. Dadurch wird das Anstechmittel repetierend zum Anstechen der Haut aus dem Nadelmodul heraus und anschließend wieder in dieses hinein bewegt.

Die Kopplung kann konstruktiv verschieden ausgeprägt sein. Die Antriebseinheit weist ein Kopplungsmittel, z.B. vorzugsweise eine Schubstange, auf. Zur Kopplung werden korrespondierende Teile von lösbaren Verbindungsmitteln als Kopplungselemente an Kopplungsmittel und Halterung ausgebildet. So kann die Halterung bzw. das Kopplungsmittel endseitig eine Kugel aufweisen, die mit einer Kalotte am Kopplungsmittel bzw. der Halterung formschlüssig verbindbar ist. Ferner kann in der Halterung (im Kopplungsmittel) ein ferromagnetisches Metall integriert sein, das von einem Magneten in der Antriebseinheit (in der Halterung) angezogen wird, wobei ein Kraftschluss entsteht. Weitere Möglichkeiten der Kopplung sind insbesondere die Verwendung von Klettbändern und anderen adhäsiven Bändern. Die wieder lösbare Verbindung bleibt während des Anstechens durch eine dynamische, oszillatorische Hin- und Herbewegung der Schubstange definierter Schwingungsweite bestehen und ist entsprechend den vorliegenden, dynamischen Kräfte zu dimensionieren.

Die Fixierung der Nadel bzw. der Nadeln an der Halterung erfolgt in an sich bekanntner Weise.

Das Nadelmodul umfasst weiter eine äußere Abdeckung, beispielsweise ein geschlossenes Gehäuse. In einer bevorzugten Ausfgestaltung ist das Gehäuse zweiteilig ausgeführt und umfasst ein Topmodul an der Spitze, in dem im wesentlichen das Anstechmittel liegt, sowie ein Grundmodul in welchem im wesentlichen die Halterung und angeordnet ist. Durch die Zweiteilung kann das Gehäuse kostengünstig mittels Spritzguss gefertigt werden.

Erfindungsgemäß ist vorzugsweise vorgesehen, dass die Abdeckung mit einem Gehäuse der Antriebseinheit überlappt, so dass der Bereich, in dem die Halterung mit der Antriebseinheit gekoppelt wird, nicht nur im Betrieb sonmdern auch bei der Kopplung nach außen/abgeschirmt ist.

Zweckmäßig ist eine Führung des Anstechmittels bzw. der Halterung ausgebildet. Die Führung kann einteilig mit der Abdeckung ausgebildet werden, oder vorzugsweise als separates Teil, welches an der Abdeckung fixiert ist. Es ist besonders bevorzugt, wenn die Führung die Halterung ringförmig umgreift. Zweckmäßig wird die Führung mit einem Wulst am äußeren Umfang in einer Nut an der Abdeckung festgelegt. Eine Führung kann aber auch anderweitig realisiert werden.

Die erfindungsgemäß vorgesehene Sicherungsvorrichtung wird beim Lösen der Kopplung von Nadelmodul und Antriebseinheit aktiv, indem eine mechanische Blockade des Anstechmittels erzeugt wird. Es ist nach Aktivierung der Sicherungsvorrichtung nicht mehr möglich, das Anstechmittel aus dem Nadelmodul herauszufahren. Damit wird eine Wiederverwendung und ein Verletzungsrisiko ausgeschlossen.

Die Sicherungsvorrichtung besteht z.B. aus einem Vorsprung und einem Sicherungselement. Von diesen beiden ist ein Teil an der Halterung und das andere Teil an der Abdeckung des Nadelmoduls so zueinander positioniert, dass das Sicherungselement vor und während dem Gebrauch in allen Stellungen des Anstechmittels von der Seite des Anstechmittels her vor dem Vorsprung liegt, und beim Abziehen des Nadelmoduls hinter den Vorsprung bewegt wird.

Das Sicherungselement ist ganz oder teilweise aus einem elastischen Material gebildet. Die Abmessungen von Vorsprung und Sicherungslement werden so abgestimmt, dass das Sicherungselement nicht ohne Kraftaufwand an dem Vorsprung vorbeibewegt werdne kann. Dabei ist die Kraft, die beim Abziehen des Nadelmoduls von der Antriebseinheit zum Bewegen des Sicherungselemntes hinter den Vorsprung notwendig ist, kleiner als die Adhäsionskraft der Kopplung. Während das Sicherungselement an dem Vorsprung vorbeibewegt wird, findet eine elastische Verformung statt, so dass es an dem Vorsprung vorbeibewegt werdne kann. Sobald es den Vorsprung passiert hat, verformt es sich zurück und kann dann ohne Kraftaufwand nicht mehr an dem Vorsprung vorbei in die Ausgangslage zurück.

Die räumliche Gestaltung von Vorsprung und Sicherungselement wird vorzugsweise so gewählt, dass der Kraftauwand für das Vorbeibewegen an dem Vorsprung geringer ist, als ein für ein Zurückbewegen notwendiger Kraftaufwand. Dies ist beispielsweise durch Federn oder Federzungen als Sicherungselement realisierbar, die in einer Richtung leicht andrückbar sind, sich aber in der Gegenrichtung aufspreizen und damit ein Zurückziehen faktisch ausschließen. Vorzugsweise ist der Kraftaufwand für das Vorbeibewegen des Sicherungselementes aus der Gebrauchsstellung hinter den Vorsprung gering.

Das elastische Sicherungselement ist in einer ersten bevorzugten Ausgestaltung eine Feder, Federzunge oder ein Widerhaken. Der Vorsprung kann z.B. ein Wulst oder Kragen sein, bevorzugt wird er von der Führung gebildet. Es ist aber auch möglich, Vorsprung und Führung als separate Teile zu gestalten.

In einer bevorzugten Ausführungsform wird das Sicherungselement von einem Widerhaken oder einer Federzunge, oder mehreren davon, an der Halterung gebildet und der Vorsprung wird von der Führung gebildet.

In einer zweiten bevorzugten Ausgestaltung wird als Sicherungselement ein Kranz von Federzungen an der Abdeckung, bevorzugt an der Führung, des Nadelmoduls vorgesehen und der Vorsprung ist ein Wulst, Kragen oder O-Ring an der Halterung.

In einer alternativen Ausgestaltung der Sicherungsvorrichtung erfolgt die mechanische Blockade dadurch, dass das Anstechmittel beim Abkoppeln von der Antriebseinheit aus seiner Austrittsöffnung in der Abdeckung des Nadelmoduls herausgezogen wird und die Halterung ein radiales Spiel aufweist. Sobald das Anstechmittel nicht mehr in der Austrittsöffnung zentriert ist, kann es nicht mehr in diese zurückgebracht werden, da die Halterung keine zum Treffen der Öffnung ausreichende Zentrierung besitzt oder sogar mittels eines elastischen Elementes oder einer nicht-zentrierten Lagerung aus dieser zentrierten Stellung gedrückt wird. Hierbei ist es bevorzugt, wenn die Abdeckung des Nadelmoduls ein Treffen der Öffnung weiter erschwert, indem diese sich an der Spitze eines mit der Spitze zur Antriebseinheit weisenden Kegels befindet.

Es ist bei dieser Ausgestaltung weiter bevorzugt, dass die Abdeckung des Nadelmoduls und die Antriebseinheit den Bereich der Kopplungselemente vor einem Zugriff schützen, damit die Halterung nicht manuell zentriert werden kann. Zweckmäßig ist daher eine ausreichender Überlapp der Gehäuse vorgesehen.

Andere Ausgestaltungen der Sicherungsvorrichtung sind denkbar, wichtig ist allein, dass durch das Abkoppeln des Nadelmoduls von der Antriebseinheit eine mechanische Blockade des Anstechmittels erzeugt wird, welche dieses irreversibel im Inneren des Nadelmoduls fixiert.

In einer bevorzugten Ausführungsform umfasst das Nadelmodul eine Arretiervorrichtung. Das Material der Arretiervorrichtung kann z.B. Kunststoff und/oder Metall sein. Vor der Benutzung ist das Anstechmittel von der Arretiervorrichtung fixiert. Wenn das Nadelmodul an die Antriebseinheit gekoppelt ist, wird die Arretiervorrichtung gelöst, so dass die Fixierung des Anstechmittels aufgehoben ist. Beispielsweise kann die Arretiervorrichtung ein Stift, Bolzen oder Steg sein, der in eine Ausnehmung an der Halterung des Anstechmittels eingreift bzw. an der Halterung befestigt ist. Zum Lösen wird der Stift/Bolzen herausgezogen bzw. der Steg entfernt, z.B. abgebrochen oder weggebogen. Damit kann die an die Antriebseinheit gekoppelte Halterung von dieser wie vorgesehen frei bewegt werden und das Anstechmittel in die Haut einstechen und wieder herausziehen. Die Arretiervorrichtung erleichtert das Aufstecken des Nadelmoduls auf die Antriebseinheit.

In einer bevorzugten Ausgestaltung ist die Arretiervorrichtung in die Führung integriert, indem die Führung an einer Stelle einen Durchbruch aufweist, durch den hindurch ein Stift/Bolzen in eine Ausnehmung an der Halterung eingreift. Durch diese Konstruktion lässt sich ein besonders einfacher Aufbau des Nadelmoduls erreichen, der auch unabhängig von der Sicherung vorteilhaft ist. Daher betrifft die Erfindung auch ein Nadelmodul, bei dem die Halterung mit einer Führung gelagert ist und die Führung zur Fixierung des Anstechmittels bis zum Gebrauch eine Arretiervorrichtung aufweist.

Die Antriebseinheit umfasst ein Kopplungsmittel, vorzugsweise eine Schubstange, zur Übertragung der Schubbewegung auf die Halterung in dem Nadelmodul. In der Regel ist ein Getriebe vorgesehen. Weiterhin bildet die Antriebseinheit typischerweise den Griff, mit dem das Gerät zum Anstechen der Haut gehalten wird.

Das Gerät umfasst weiterhin eine Energieversorgung. Üblicherweise wird in der Antriebseinheit ein Antrieb, insbesondere ein Elektromotor, vorgesehen, der die repetierende Schubbewegung erzeugt. Die Antriebseinheit wird dabei, nötigenfalls über ein Netzteil, durch ein Netzkabel mit Strom versorgt, oder mit einem Akkumulator oder einer Batterie betrieben.

Typischerweise sind Ein-/Ausschalter und Regeleinrichtungen für die Repetiergeschwindikgeit und/oder die Einstechtiefe vorhanden. Schalter und Regler können in der Antriebseinheit oder in einem Steuergerät untergebracht sein. Ist ein Steuergerät vorgesehen, wird ein etwa nötiges Netzteil vorzugsweise in dieses integriert. Bevorzugt befindet sich der Ein-/Ausschalter an der Antriebseinheit. Er kann aus Sicherheitsgründen als Druckschalter ausgelegt sein, so dass die Schubbewegung nur solange bereitgestellt bzw. auf das Anstechmittel übertragen wird, wie der Bediener den Schalter gedrückt hält.

Nadelmodul und Antriebseinheit können in an sich bekannter Weise gegen den Gebrauch ungeeigneter Kombinationen der beiden gesichert sein, sofern nicht die spezifische Kopplung bereits die Verwendung eines für die Antriebseinheit nicht vorgesehenen Nadelmoduls verhindert.

In einer Ausführungsform, insbesondere bei Anwendung im Bereich Medizin, kann ein Reservoir für einen in die Haut zu applizierenden Stoff im Nadelmodul integriert sein. Dies ist z.B. für Impfungen vorteilhaft. Aber auch zur Applikation von Permanent Make-up kann es vorteilhaft sein, dass eine übliche benötigte Menge Pigment in einem Reservoir im Nadelmodul vorliegt.

Die Erfindung soll anhand der beigefügten Figuren näher erläutert werden, ohne jedoch auf die speziell beschriebenen Ausführungsformen beschränkt zu sein. Die Erfindung bezieht sich auch auf sämtliche Kombinationen von bevorzugten Ausgestaltungen, soweit diese sich nicht gegenseitig ausschließen. Die Angaben "etwa" oder "ca." in Verbindung mit einer Zahlenangabe bedeuten, dass zumindest um 10 % höhere oder niedrigere Werte oder um 5 % höhere oder niedrigere Werte und in jedem Fall um 1 % höhere oder niedrigere Werte eingeschlossen sind.

Dabei zeigt:
Fig. 1 schematisch den Aufbau eines Handgerätes mit aufgesetztem Nadelmodul
Fig. 2 ein Detail der Kopplung (Magnet und ferromagnetisches Metall)
Fig. 3 Querschnitte des Nadelmoduls entlang der Linien A-A und B-B
Figur 4 alternative Kopplungsvarianten
Fig. 5 verschiedene Positionen für eine Arretiervorrichtung
Fig. 6 a bis d verschiedene Ausgestaltungen für Arretiervorrichtungen
Fig. 7 a bis c das Handgerät vor dem Gebrauch, die Änderungen zum Gebrauch und das Nadelmodul nach dem Gebrauch
Fig. 8 ein Sicherungselement im Detail.

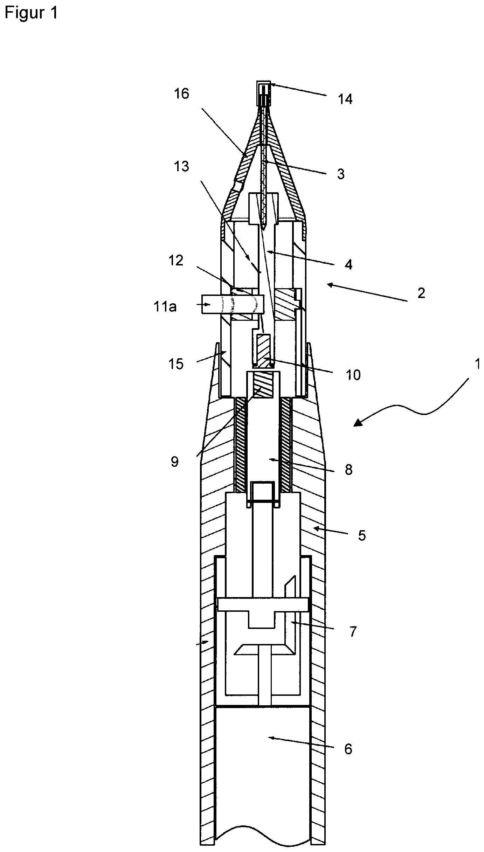

In Figur 1 ist der Aufbau eines Handgerätes 1 schematisch gezeigt. Das Nadelmodul 2 umfasst eine Nadel als Anstechmittel 3, die in einer Halterung 4 aufgenommen ist. Die Antriebseinheit 5 umfasst einen Motor 6, welcher über ein Getriebe 7 und ein Kopplungsmittel 8 in Form einer Schubstange mit der Halterung 4 lösbar koppelbar ist. Die Kopplung der Halterung 4 an die Antriebseinheit 5 wird durch einen Magneten 9 in der Schubstange 8 und ein ferromagnetisches Material 10 in der Halterung 4 bewirkt.

Weiterhin weist das Nadelmodul 2 eine Führung 12 auf, die zugleich Teil einer Arretiervorrichtung 11 ist, indem die Führung 12 einen Durchbruch 11a hat, in welchem ein Bolzen 11b angeordnet ist, der in eine Ausnehmung 11c an der Halterung 4 eingreifen kann. Mit Hilfe des Bolzen 11a wird die Halterung 4 bis zum Gebrauch fixiert. In der gezeigten Ausgestaltung erfolgt die Kopplung auch erst endgültig, wenn der Bolzen 11a aus der Ausnehmung 11c gezogen wird. Erst dann kann die Halterung 4 mit der Schubstange 8 in direkten Kontakt treten.

Die Sicherungsvorrichtung besteht aus einem Vorsprung, der von der Führung 12 gebildet wird, und dem Sicherungselement 13. Die Nadel 3 wird bis zum Gebrauch von der Schutzkappe 14 abgedeckt. Weiter ist zu erkennen, dass das Nadelmodul 2 in diesem Fall eine zweiteilig ausgeführte Abdeckung hat, die sich aus Grundmodul 15 und Topmodul 16 zusammensetzt.

Die Nadel 3 ist üblicherweise fest und unlösbar mit der Halterung 4 verbunden. Der Bediener wählt zu Beginn einer Behandlung ein Nadelmodul 2 mit einer gewünschten Nadelgeometrie und -durchmesser aus. Es werden während einer Behandlung meistens mehrere Nadelmodule 2 mit unterschiedlichen Nadeln 3 verwendet.

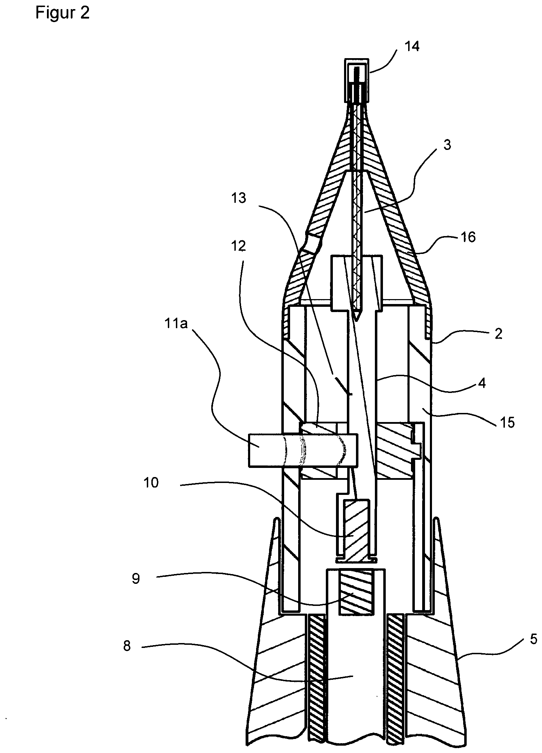

In Fig. 2 ist das mit der Antriebseinheit 5 gekoppelte Nadelmodul 2 nochmal vergrößert gezeigt. Die Halterung 4 beinhaltet ein Kopplungselement, das eine einfache, wieder lösbare Verbindung mit der Schubstange 8 der Abtriebseinheit 5 ermöglicht. Konstruktive Realsierungen des Kopplungselements sind beispielsweise formschlüssige Schnappverbindungselemente, Klemm- und Klebeverbindungen, Klettbänder oder Magnetelemente.

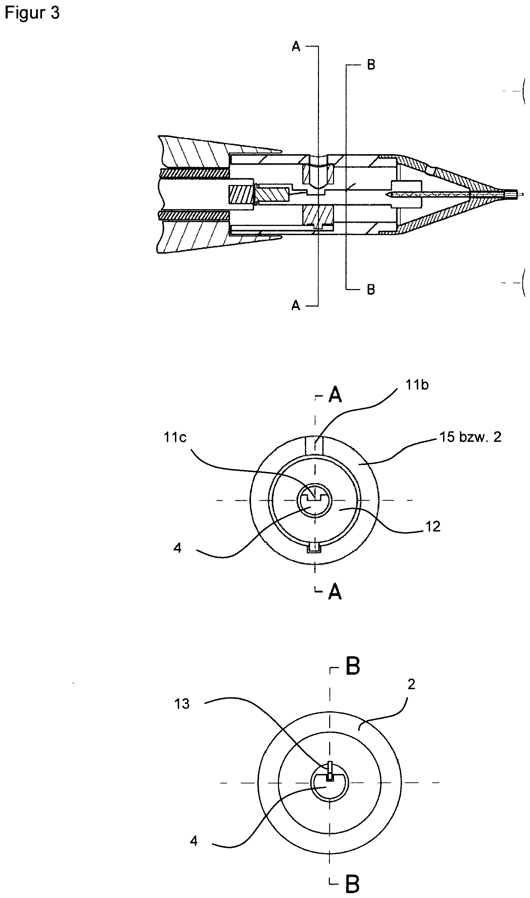

In Figur 3 ist oben nochmals das Nadelmodul 2 gezeigt, wobei Linien A-A und B-B eingezeichnet sind. Die beiden Querschnitte entlang dieser Linien sind unten in Figur 3 zu sehen. Im Schnitt entlang A-A ist zu erkenne, dass die Führung 12 und die Abdeckung 15 des Nadelmoduls 2 als Teil der Arretiervorrichtung 11 einen Durchbruch 11b aufweisen, den der in Figur 2 gezeigte Bolzen 11a durchgreifen kann um dann in die Ausnehmung 11c der Halterung 4 einzugreifen und die Halterung 4 so zu fixieren.

In Figur 3 ist weiter zu erkennen, dass die Halterung 4 in der Führung 12, die auch den Vorsprung für die Sicherungsvorrichtung bildet, geführt wird. Die Halterung ist im Bereich zwischen dem Kopplungselement 10 und der Fixierung des Anstechmittels 3 abgeflacht. Wie in dem Schnitt B-B zu erkennen, erleichtert dies die Dimensionierung des Sicherungselementes 13 und gibt diesem Raum für seine elastische Verformung während es an der Führung 12 (die den Vorsprung bildet) vorbeigezogen wird. Alternativ zu der Abflachung könnte z.B. eine Rille vorgesehen sein, in welche sich das Sicherungselement verformen kann.

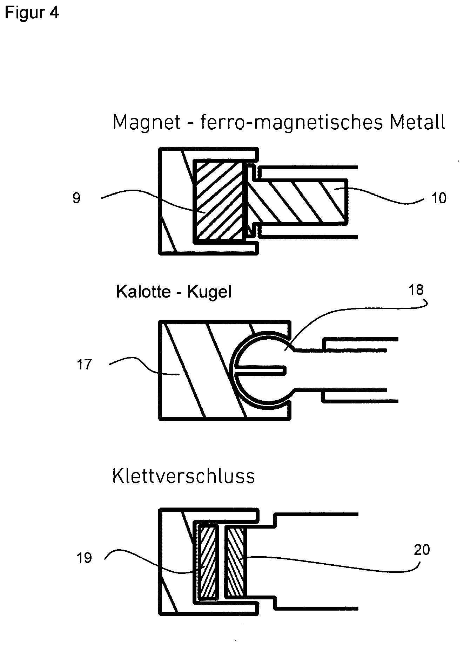

Fig. 4 stellt verschiedene Beispiele von Kopplungselementen für das erfindungsgemäße Gerät 1 dar. In Fig. 4a ist nochmals die magnetische Kopplung gezeigt, wobei natürlich auch der Magnet in der Halterung und das magnetische Material an der Schubstange vorliegen kann. Fig. 4b veranschaulicht eine Kopplung über Kalotte 17 und Kugel 18. Gemäß Fig. 4c werden die korrespondierenden Teile Hakenkomponente 19 und Schlingenkomponente 20 eines Klettverschlusses an Halterung und Antriebseinheit vorgesehen. Auch diese Kopplungsmittel können natürlich reziprok angeordnet sein.

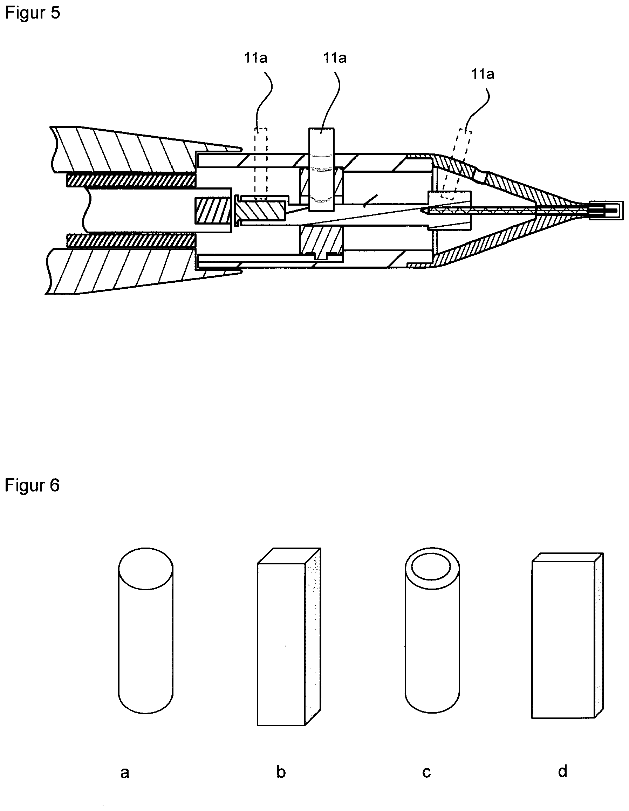

Figur 5 zeigt, wie der geometrische Ort der Arretiervorrichtung 11 in Bewegungsrichtung beliebig wählbar ist. Fig. 6 a bis d stellt Beispiele für Ausformungen von Arretiervorrichtungen 11 schematisch dar. Die Vorrichtung 11 soll dabei eine ausreichende mechanische Festigkeit in Material und Verformung (Biegesteifigkeit) besitzen, damit während des Aufsteckens des Nadelmoduls 2 auf die Antriebseinheit 5 die Halterung 4 in ihrer Ausgangslage bleibt.

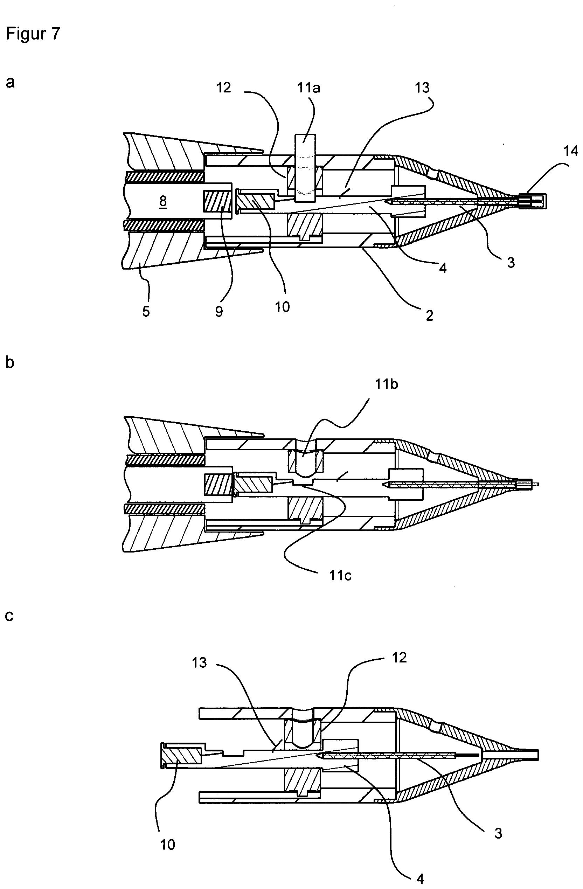

In Figur 7a bis c ist das Nadelmodul 2 während und nach der Behandlung gezeigt. Nach dem Aufstecken des Nadelmoduls 2 und vor dem Beginn der Behandlung wird die Arretiervorrichtung 11 gelöst, d.h. der Bolzen 11a herausgezogen, wie in Figur 7a veranschaulicht. Nach diesem Entsperren zieht der Magnet 9 der Schubstange 8 der Antriebseinheit 5 die Halterung 4 mit dem magentischen Material 10 an und bildet eine kraftschlüssige Verbindung.

Die Halterung 4 wird in einer so genannten Führung 12 geführt, wobei die Arretiervorrichtung 11 wie gezeigt in die Führung 12 integriert sein kann. Die Führung 12 gewährleistet eine weitestgehend geradlinige, geführte Hin- und Herbewegung der Halterung 4 mit der Nadel 3. Ein Knicken / Abweichen der Nadel 3 aus der Bewegungsrichtung unter Last wird damit verhindert. Die Führung 12 kann an einem beliebigen Ort entlang der Bewegungsachse der Halterung 4 angeordnet sein. Idealerweise befindet sich die Führung 12 mittig im Bereich des Halterung 4, da diese dort, bedingt durch ihre Aufgaben als Nadelhalter, eine ausreichende mechanische Biegesteifigkeit und Festigkeit besitzt.

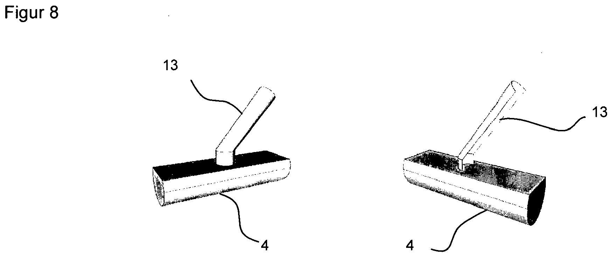

In der gezeigten Ausführungsform besitzt die Führung 12 zusammen mit dem Sicherungselement 13 erfindungsgemäß auch die Aufgabe der irreversiblen Bewegungseinschränkung der Nadel 3 mit Halterung 4 nach dem Trennen von Nadelmodul 2 und Antriebseinheit 5. Dies geschieht durch eine spezifisch gewählte Anordnung von Führung 12 und Sicherungselement 13. Das Sicherungselement 13 ist insbesondere aus Kunststoff oder Metall gefertigt und kann ein-oder mehrteilig, insbesondere als Fächerelement ausgeformt sein. Fig. 8 zeigt ein Beispiel.

Das Sicherungselement 13 ist dergestalt in Richtung der Bewegungsachse angeordnet, dass es zum einen die Hin- und Herbewegung der Nadel 3 nach dem Koppeln des Nadelmoduls 2 mit der Antriebseinheit 5, d.h. während der Behandlung, z.B. des Farbstoffeintrags, nicht behindert. Zum anderen lässt es nach dem Trennen von Nadelmodul 2 und Antriebseinheit 5, d.h. nach Beendigung einer Behandlung, die Nadel 3 irreversibel und vollständig im Nadelmodul 2 eintauchen und hält sie dort fest. Ein Herausbewegen der Nadel 3 aus dem Nadelmodul 2 ist nicht mehr möglich. Fig. 7c zeigt diesen Zustand.

Insbesondere bewegt sich das Sicherungselement 13 während des oben beschriebenen Trennvorgangs durch die Führung 12 hindurch. Dabei verformt es sich so, dass eine erneute Bewegungsrichtung der Nadel 3 in die Ausgangslage konstruktiv verhindert wird.

### Bezugszeichenliste

- 1: Gerät, insbesondere Handgerät
- 2: Nadelmodul
- 3: Anstechmittel, d.h. Nadel oder Nadelsystem
- 4: Halterung
- 5: Antriebseinheit
- 6: Motor
- 7: Getriebe
- 8: Kopplungsmittel, insbesondere Schubstange
- 9: Kopplungselement Magnet
- 10: Kopplungselement magn. Material
- 11: Arretiervorrichtung mit 11a Bolzen, 11b Durchbruch und 11c Ausnehmung
- 12: Führung, auch Vorsprung als Teil der Sicherungsvorrichtung
- 13: Sicherungselement
- 14: Schutzkappe
- 15: Grundmodul
- 16: Topmodul
- 17: Kalotte als Kopplungselement
- 18: Kugel als Kopplungselement
- 19: Hakenkomponente als Kopplungselement
- 20: Schlingenkomponente als Kopplungselement

## Patentansprüche

1. Nadelmodul (2), umfassend ein Anstechmittel (3) an einer Halterung (4), welche mit einer Antriebseinheit (5) lösbar koppelbar ist, so dass durch eine von der Antriebseinheit (5) auf die Halterung (4) ausgeübte Antriebskraft eine repetierende Schubbewegung zum Ein- und Ausfahren des Anstechmittels (3) übertragen wird, **dadurch gekennzeichnet, dass** die Halterung (4) eine Sicherungsvorrichtung (12, 13) aufweist, welche das Anstechmittel (4) nach dem Lösen des Nadelmoduls (2) von der Antriebseinheit (5) so mechanisch irreversibel blockiert, dass es nicht mehr aus dem Nadelmodul (3) ausgefahren werden kann.

2. Nadelmodul gemäß Anspruch 1, **dadurch gekennzeichnet, dass** es als Anstechmittel (3) eine Nadel aufweist, oder ein Nadelsystem mit zwei, drei oder mehr Nadeln.

3. Nadelmodul gemäß Anspruch 1 oder 2, **dadurch gekennzeichnet, dass** die Sicherungsvorrichtung ein Sicherungselement (13) aus einem elastischen Material und einen Vorsprung (12) umfasst.

4. Nadelmodul gemäß Anspruch 3, **dadurch gekennzeichnet, dass** der Vorsprung von einer Führung (12) gebildet wird, das Sicherungselement (13) die Form einer Federzunge, eines Widerhakens oder eines Fächerelements aufweist und so an der Halterung (4) positioniert ist, dass es beim Abziehen des Nadelmoduls (2) von der Antriebseinheit (5) an dem Vorsprung (12) vorbeigezogen wird und der Vorsprung (12) ein Zurückbewegen blockiert.

5. Nadelmodul gemäß Anspruch 3, **dadurch gekennzeichnet, dass** das Sicherungselement (13) eine Feder oder ein Kranz von Federzungen oder Widerhaken an der Abdeckung (15, 16) des Nadelmoduls (2) ist und der Vorsprung (13) ein Wulst, Kragen oder O-Ring an der Halterung (4) ist.

6. Nadelmodul gemäß mindestens einem der Ansprüche 1 bis 5, **dadurch gekennzeichnet, dass** es eine Arretiervorrichtung (11) aufweist, welche die Halterung (4) bis zum Gebrauch in einer Ausgangslage innerhalb des Nadelmoduls (2) fixiert.

7. Nadelmodul gemäß mindestens einem der Ansprüche 1 bis 6, **dadurch gekennzeichnet, dass** es eine Führung (12) umfasst, die eine im wesentlichen geradlinige, geführte Hin- und Herbewegung der Halterung (4) mit des Anstechmittels (3) gewährleistet.

8. Nadelmodul (2) gemäß mindestens einem der Ansprüche 1 bis 7, **dadurch gekennzeichnet, dass** die Halterung (4) eine Führung (12) mit einer integrierten Arretiervorrichtung (11a, 11b, 11c) umfasst, wobei die Führung (12) zusammen mit einer Austrittsöffnung für das Anstechmittel (3) in einer Abdeckung (15, 16) des Nadelmoduls (2) eine im wesentlichen geradlinige, geführte Hin- und Herbewegung der Halterung (4) mit dem Anstechmittel (3) gewährleistet und die Arretiervorrichtung die Halterung (4) bis zum Gebrauch in einer Ausgangslage innerhalb des Nadelmoduls (2) fixiert.

9. Nadelmodul gemäß Anspruch 8, **dadurch gekennzeichnet, dass** die Halterung (4) ein radiales Spiel aufweist und das Anstechmittel (3) beim Lösen des Nadelmoduls (2) von der Antriebseinheit (5) in das Innere der Abdeckung (15, 16) des Nadelmoduls (2) gezogen wird, so dass es aufgrund des radialen Spiels der Halterung (4) nicht wieder durch die Austrittsöffnung nach außen treten kann.

10. Nadelmodul gemäß Anspruch 9, **dadurch gekennzeichnet, dass** die Halterung (4) mittels eines elastischen Elementes oder einer nicht-zentrierten Lagerung nach dem Lösen des Nadelmoduls (2) von der Antriebseinheit (5) aus einer zentrierten Stellung gedrückt ist.

11. Nadelmodul gemäß Anspruch 8 oder 10, **dadurch gekennzeichnet, dass** an der Spitze der Abdeckung (15, 16) des Nadelmoduls (2) ein mit der Spitze zur Antriebseinheit (5) weisender Kegel ausgebildet ist.

12. Nadelmodul gemäß Anspruch 10 oder 11, **dadurch gekennzeichnet, dass** die Abdeckung (15, 16) des Nadelmoduls (2) zweiteilig ausgeführt ist und aus einem Grundmodul (15), in welchem die Halterung (4) angeordnet ist, und einem Topmodul (16) an der Spitze, in dem das Anstechmittel (3) liegt, besteht.

13. Gerät (1), insbesondere Handgerät, zum Anstechen der Haut, umfassend eine Antriebseinheit (5) und ein Nadelmodul (2) gemäß einem der Ansprüche 1 bis 7, wobei die Antriebseinheit (5) einen Motor (6) und Kopplungsmittel (8) aufweist.

14. Gerät gemäß Anspruch 13, **dadurch gekennzeichnet, dass** die Antriebseinheit (5) als Kopplungsmittel (8) eine Schubstange aufweist.

15. Gerät gemäß Anspruch 14, **dadurch gekennzeichnet, dass** zur Kopplung von Antriebseinheit (5) und Nadelmodul (2) korrespondierende Teile von lösbaren Verbindungsmitteln an Kopplungsmittel (8) und Halterung (4) ausgebildet werden.

16. Gerät gemäß Anspruch 15, **dadurch gekennzeichnet, dass** die korrespondierenden Teile von lösbaren Verbindungsmitteln ausgewählt sind unter Kugel (18) und Kalotte (17), Magnet (9) und ferromagnetisches Material (10), Haken- und Schlingenkomponente (19, 20).

## Claims

1. Needle module (2) comprising a piercing means (3) on a mounting (4), which can be releasably coupled to a drive unit (5) so that, as a result of a driving force exerted by the drive unit (5) on the mounting (4), a repeated pushing movement moving the piercing means (3) in and out is transmitted, **characterized in that** the mounting (4) has a safety device (12, 13) which mechanically irreversibly blocks the piercing means after the needle module has been released from the drive unit (5) so that it can no longer be moved out of the needle module (3).

2. Needle module according to claim 1, **characterized in that**, as a piercing means (3), it has a needle or a needle system with two, three or more needles.

3. Needle module according to claim 1 or 2, **characterized in that** the safety device comprises a safety element (13) of an elastic material and a projection (12).

4. Needle module according to claim 3, **characterized in that** the projection is formed by a guide (12), the safety element (13) is in the form of a flexible tongue, a barb or a fan element and is positioned on the mounting (4) so that, when the needle module (2) is withdrawn from the drive unit (5), it is drawn past the projection (12) and the projection (12) blocks any backwards movement.

5. Needle module according to claim 3, **characterized in that** the safety element (13) is a spring or a ring of flexible tongues or barbs on the cover (15, 16) of the needle module (2) and the projection (13) is a bulge, collar or O-ring on the mounting (4).

6. Needle module according to at least one of claims 1 to 5, **characterized in that** it comprises a locking mechanism (11) which fixes the mounting (4) in an initial position within the needle module (2) until use.

7. Needle module according to at least one of claims 1 to 6, **characterized in that** it comprises a guide (12) which guarantees an essentially straight, guided back-and-forth movement of the mounting (4) with the piercing means (3).

8. Needle module (2), according to at least one of claims 1 to 7, **characterized in that** the mounting (4) comprises a guide (12) with an integrated locking mechanism (11a, 11b, 11c), wherein the guide (12) together with an outlet opening for the piercing means (3) in a cover (15, 16) of the needle module (2) guarantees an essentially straight, guided back-and-forth movement of the mounting (4) with the piercing means (3) and the locking mechanism fixes the mounting (4) in an initial position within the needle module (2) until use.

9. Needle module according to claim 8, characterized the mounting (4) has a radial play and, when the needle module (2) is released from the drive unit (5), the piercing means (3) is drawn into the inside of the cover (15, 16) of the needle module (2) so that it cannot move back out through the outlet opening owing to the radial play of the mounting (4).

10. Needle module according to claim 9, **characterized in that** the mounting (4), by means of an elastic element or non-centred bearing, is forced out of a centred position after releasing the the needle module (2) from the drive unit (5).

11. Needle module according to claim 8 or 10, **characterized in that** a cone with its tip pointing towards the drive unit (5) is formed on the tip of the cover (15, 16) of the needle module (2).

12. Needle module according to claim 10 or 11, **characterized in that** the cover (15, 16) of the needle module (2) is made two part and consists of a base module (15) in which the mounting (4) is arranged and a top module (16) at the tip, in which the piercing means (3) lies.

13. Device (1), in particular hand-held device, for piercing the skin, comprising a drive unit (5) and a needle module (2) according to one of claims 1 to 7, wherein the drive unit (5) comprises a motor (6) and coupling means (8).

14. Device according to claim 13, **characterized in** the drive unit (5) has a push rod as coupling means (8).

15. Device according to claim 14, **characterized in that**, for the coupling of drive unit (5) and needle module (2), corresponding parts of releasable connection means are formed on coupling means (8) and mounting (4).

16. Device according to claim 15, **characterized in that** the corresponding parts of releasable connection means are selected from ball (18) and socket (17), magnet (9) and ferromagnetic material (10), hook and loop components (19, 20).

## Revendications

1. Module d'aiguille (2) comprenant un moyen de piqûre (3) à un support (4), lequel peut être couplé de manière détachable à une unité d'entraînement (5) de sorte que par une force d'entraînement exercée par l'unité d'entraînement sur le support (4), un mouvement répétitif de poussée d'entrée et de sortie du moyen de piqûre (3) est transmis, **caractérisé en ce que** le support (4) présente un dispositif de sécurité (12, 13) qui bloque le moyen de piqûre (4) de manière mécaniquement irréversible, après le détachement du module d'aiguille (2) de l'unité d'entraînement (5) de sorte qu'il ne peut plus être sorti du module d'aiguille (3).

2. Module d'aiguille selon la revendication 1, **caractérisé en ce qu'**il présente comme moyen de piqûre (3) une aiguille ou un système d'aiguille avec deux ou trois aiguilles ou davantage.

3. Module d'aiguille selon la revendication 1 ou 2, **caractérisé en ce que** le dispositif de sécurité comprend un élément de sécurité (13) en matière élastique et une saillie (12).

4. Module d'aiguille selon la revendication 3, **caractérisé en ce que** la saillie est formée d'un guide (12), l'élément de sécurité (13) présente la forme d'une languette à ressort, d'un ardillon ou d'un élément en éventail et ainsi est positionné au support (4) de sorte qu'en cas de retrait du module d'aiguille (2) de l'unité d'entraînement (5) il est tiré à la saillie (12) et la saillie (12) bloque un mouvement de retour.

5. Module d'aiguille selon la revendication 3, **caractérisé en ce que** l'élément de sécurité (13) est un ressort ou une crête de languettes à ressort ou des ardillons au recouvrement (15, 16) du module d'aiguille (2) et la saillie (13) est un renflement, un collet ou un joint torique au support (4).

6. Module d'aiguille selon au moins l'une des revendications 1 à 5, **caractérisé en ce qu'**il présente un dispositif d'arrêt (11) qui fixe le support (4) jusqu'à l'usage dans une position de repos à l'intérieur du module d'aiguille (2).

7. Module d'aiguille selon au moins l'une des revendications 1 à 6, **caractérisé en ce qu'**il comprend un guide (12) qui garantit un mouvement guidé du support (4) de va-et-vient et essentiellement rectiligne avec le moyen de piqûre (3).

8. Module d'aiguille (2) selon au moins l'une des revendications 1 à 7, **caractérisé en ce que** le support (4) comprend un guide (12) avec un dispositif d'arrêt (11a, 11b, 11c) intégré, le guide (12) garantissant, avec une ouverture de sortie pour le moyen de piqûre (3) dans un recouvrement (15, 16) du module d'aiguille (2) un mouvement guidé du support (4) de va-et-vient et essentiellement rectiligne avec le moyen de piqûre (3) et le dispositif d'arrêt fixant le support (4) jusqu'à l'usage dans une position de repos à l'intérieur du module d'aiguille (2).

9. Module d'aiguille selon la revendication 8, **caractérisé en ce que** le support (4) présente un jeu radial et le moyen de piqûre (3) est tiré en cas de détachement du module d'aiguille (2) de l'unité d'entraînement (5) dans l'intérieur du recouvrement (15, 16) du module d'aiguille (2) de sorte qu'en raison du jeu radial du support (4), il ne puisse pas à nouveau sortir par l'ouverture de sortie.

10. Module d'aiguille selon la revendication 9, **caractérisé en ce que** le support (4) est poussé, au moyen d'un élément élastique ou d'une position non centrée après le détachement du module d'aiguille (2) de l'unité d'entraînement (5), d'une position centrée.

11. Module d'aiguille selon la revendication 8 ou 10, **caractérisé en ce qu'**à la pointe du recouvrement (15, 16) du module d'aiguille (2) est configuré un cône pointant avec la pointe vers l'unité d'entraînement (5).

12. Module d'aiguille selon la revendication 10 ou 11, **caractérisé en ce que** le recouvrement (15, 16) du module d'aiguille (2) est exécuté en deux parties et est constitué d'un module fondamental (15) dans lequel est agencé le support (4) et d'un module de sommet (16) à la pointe dans lequel se trouve le moyen de piqûre (3).

13. Appareil (1), en particulier appareil portatif, pour piquer la peau, comprenant une unité d'entraînement (5) et un module d'aiguille (2) selon l'une des revendications 1 à 7, l'unité d'entraînement (5) présentant un moteur (6) et un moyen de couplage (8).

14. Appareil selon la revendication 13, **caractérisé en ce que** l'unité d'entraînement (5) présente comme moyen de couplage (8) une tige de poussée.

15. Appareil selon la revendication 14, **caractérisé en ce que** pour le couplage de l'unité d'entraînement (5) et du module d'aiguille (2) des pièces correspondantes de moyens de raccord détachables sont configurées aux moyens de couplage (8) et support (4).

16. Appareil selon la revendication 15, **caractérisé en ce que** les pièces correspondantes des moyens de raccord détachables sont sélectionnées parmi une bille (18) et une pièce concave (17), un aimant (9) et un matériau ferromagnétique (10), un composant en crochets et boucles (19, 20).
